# EUROPEAN PATENT SPECIFICATION

(11) **EP 3 989 948 B1**
(45) Date of publication and mention of the grant of the patent: **30.09.2026**
(21) Application number: 20736948.9
(22) Date of filing: 26.06.2020
(51) Int. Cl.: A61K 9/00, A61K 9/10, A61K 33/38

(54) **PHARMACEUTICAL COMPOSITION COMPRISING A COLLOIDAL DISPERSION AND METHODS AND USES THEREOF**
PHARMAZEUTISCHE ZUSAMMENSETZUNG MIT EINER KOLLOIDALEN DISPERSION UND VERFAHREN UND VERWENDUNGEN DAVON
COMPOSITION PHARMACEUTIQUE COMPRENANT UNE DISPERSION COLLOÏDALE ET MÉTHODES ET UTILISATIONS ASSOCIÉES

(30) Priority: 26.06.2019 EP 19182655
(43) Date of publication of application: 04.05.2022
(73) Proprietor: PREBONA AB, 272 36 Simrishamn (SE)
(72) Inventor: ÖSTBERG, Christian, 181 66 Lidingö (SE)
(74) Representative: Brann AB
(86) International application number: PCT/EP2020/068113
(87) International publication number: WO 2020/260643

(56) References cited:
- WO-A1-2011/037523
- DANA BARAM-PINTO ET AL: "Inhibition of Herpes Simplex Virus Type 1 Infection by Silver Nanoparticles Capped with Mercaptoethane Sulfonate", BIOCONJUGATE CHEMISTRY, AMERICAN CHEMICAL SOCIETY, US, vol. 20, no. 8, 19 August 2009 (2009-08-19), pages 1497 - 1502, XP002622422, ISSN: 1043-1802, [retrieved on 20090708], DOI: 10.1021/BC900215B
- HUMBERTO H LARA ET AL: "Silver nanoparticles are broad-spectrum bactericidal and virucidal compounds", JOURNAL OF NANOBIOTECHNOLOGY, BIOMED CENTRAL, GB, vol. 9, no. 1, 3 August 2011 (2011-08-03), pages 30, XP021105520, ISSN: 1477-3155, DOI: 10.1186/1477-3155-9-30
- STEFANIA GALDIERO ET AL: "Silver Nanoparticles as Potential Antiviral Agents", MOLECULES, vol. 16, no. 10, 24 October 2011 (2011-10-24), pages 8894 - 8918, XP055492993, DOI: 10.3390/molecules16108894

## Description

### TECHNICAL FIELD OF THE INVENTION

The present invention relates to the field of pharmaceutical compositions comprising colloidal dispersions of particles of silica to which particles silver ions have been adsorbed, which compositions are useful as antiviral agents. It is also related to a kit of parts involving said pharmaceutical compositions, optionally in combination with a further antiviral agent.

### BACKGROUND OF THE INVENTION

Nanotechnologies are the science and business of manipulating matter at the atomic scale. Materials produced with the aid of various kinds of nanotechnologies are starting to be used in many areas of everyday life such as medicine, cosmetics, clothing fabrics, sports equipment, paints, packaging, food, etc. and have been used for some time as for instance catalysts in many important industrial processes. In the future we will no doubt see many more application of nanomaterials in general and of nanomaterials involving noble metals in particular.

WO 2011/037523 A1 discloses colloidal nano-composites of silver having a well controlled size and a high degree of dispersion of silver. It also relates to processes for making such materials and using them in different applications where a biocidal effect is desired.

WO 2008/024422 A2 discloses incorporation of colloidal silver in compositions for use in partially or fully decontaminating surfaces which have been contaminated with chemical or biological warfare agents as well as to methods for treating viral infections, bacterial infections, fungal infections, and cancerous tissue.

In WO 2008/147427 A2, a composition is disclosed comprising silver particles having an interior of elemental silver and an exterior of ionic silver oxide, wherein the silver particles are present in the water at a level of about 5-40 ppm, for use in the treatment of avian influenza virus.

Dana Baram-Pinto et al., in "Inhibition of Herpes Simplex Virus Type 1 Infection by Silver Nanoparticles Capped with Mercaptoethane Sulfonate" (Bioconjugate Chemistry, Vol. 20, No. 8, 2009, pages 1497-1502), describe the use of silver nanoparticles capped with mercaptoethane sulfonate (Ag-MES) for treating viral infection, in particular Herpes Simplex Virus Type 1 Infection.

Humberto H Lara et al., in "Silver nanoparticles are broad-spectrum bactericidal and virucidal compounds" (Journal of Nanobiotechnology, Vol. 9, No. 1, 2011, page 30), present a review of recent advances in the understanding of biocidal mechanisms of action of silver nanoparticles.

Stefania Galdiero et al., in "Silver Nanoparticles as Potential Antiviral Agents" (Molecules, Vol. 16, No. 10, 2011, pages 8894-8918) present a review of silver nanoparticles as potential antiviral agents and provide a summary of different virus families and any available results of silver nanoparticle treatments on infections with these viruses.

Viral diseases are of increasing concern and, while there are more than 200 known virus species known to infect humans, there still are new species being continuously discovered (Woolhouse M, Scott F, Hudson Z, Howey R, Chase-Topping M. Human viruses: discovery and emergence. Philos Trans R Soc Lond B Biol Sci. 2012; 367(1604): 2864-2871). Examples of virus families containing pathogenic viruses are the Herpesviridae and the Flaviviridae families.

Herpesviridae is a large family of DNA viruses that cause infections in animals, including humans. Members of this family include well-known and wide-spread pathogenic species such as Herpes simplex virus-1 and 2 (HSV-1 and HSV-2) varicella zoster virus, Epstein-Barr virus, and cytomegalovirus.

Following a primary infection with herpes simplex or varicella-zoster virus, the virus establishes latency in the sensory nerve cells for the rest of the patient's life and subsequently repeated virus reactivation can occur. Following a reactivation in the nerve cell the virus is transported through the nerves to the skin, where a lesion will develop. Immediately upon an outbreak of virus replication inflammation will follow. The inflammation contributes to the symptoms associated with herpes virus recurrence, including redness, swelling, itching, and pain as well as blistering and lesions.

Herpes simplex viruses may be grouped into two serotypes, HSV type 1 (HSV-1 ) and type 2 (HSV-2), the clinical manifestations of which range from benign self-limiting orofacial and genital infections to potentially life threatening conditions such as encephalitis and generalized neonatal infections. Orofacial HSV infections are primarily caused by HSV- 1, which becomes latent after a primary infection e.g. in childhood. After reactivation a recurrent oral-facial HSV infection develops, more commonly known as a cold sore. About half of herpes infected patients experience early symptoms, e.g. pain, burning or itching at the site of the subsequent lesions. The condition is generally rapidly self-limiting and the healing time of a typical episode is about 10 days from the initial symptoms. Viral replication in the lip is initiated early and maximum virus load is attained 24 hours after the onset of the reactivation. The virus concentration is then dramatically reduced and typically virus cannot be isolated 70-80 hours after the onset.

The clinical presentation of genital HSV infections is similar to the orofacial infections with some important exceptions. Genital HSV infections are most often caused by HSV-2 and following the primary infection the virus will latently infect sensory or autonomic ganglions. Reactivation will produce the local recurrent lesions on or near the genitals that are characteristic of the herpes infection.

A primary infection with varicella-zoster virus (VZV) causes chicken-pox. Like HSV, VZV becomes latent following the primary infection and can be activated as herpes zoster later on in life. The infection usually results in skin rash and intense acute pain. In 30% of the patients, the pain can be prolonged and continue for weeks or months after the rash has cleared up, or may even be permanent.

HSV and VZV may, in addition to mucous or cutaneous manifestations, also cause keratitis in the eyes. This condition is also recurrent and may cause blindness.

There are a number of antiviral agents which are active against the human herpes viruses. However, so far clinical success in the treatment of recurrent herpes virus infections has been only limited. Thus, antivirals such as acyclovir (aciclovir), valacyclovir (valacyclovir), famciclovir, and penciclovir are used with varying success. For example, a cream formulation of acyclovir for topical application is sold by Ranbaxy under the trademark Zovirax^{®}.

Many herpes patients, however, still find that relief is unsatisfactory with presently commercially available products. Thus, there remains a need for new antiviral agents for the treatment of herpes infections.

Hepacivirus (HCV), pegiviruses, pestiviruses and flaviviruses belong to the Flaviviridae family of viruses (Rice, C. M., Flaviviridae: The viruses and their replication. In: Fields Virology, Editors: Fields, B. N., Knipe, D. M., and Howley, P. M., Lippincott-Raven Publishers, Philadelphia, Pa., Chapter 30, 931-959, 1996), which family includes a large number of pathogenic viruses. For example, the pestivirus genus includes bovine viral diarrhea virus (BVDV), classical swine fever virus (CSFV, also called hog cholera virus) and border disease virus (BDV) of sheep. The flavivirus genus includes e.g. the dengue hemorrhagic fever viruses (DHF), yellow fever virus, and Japanese encephalitis virus.

An important member of the *Flaviviridae* family is the hepacivirus genus, which includes the hepatitis C virus (HCV). More than 170 million people worldwide are affected by HCV, which is one of the major causes of severe liver disease. Around 80% of the infected individuals establish persistent infection. Around 10% of these patients develop cirrhosis. The HCV infection may proceed to development of liver cancer (e.g. hepatocellular carcinoma (HCC)), which happens in around 5% of chronic HCV carriers.

The current standard of care for treatment of *Flaviviridae* infection is limited to treatment with interferon or a combination of interferon and ribavirin. New generations of molecules, named direct-acting antivirals (DAAs) have been introduced which specifically block viral enzymes or interrupt the replication machinery. However, DAAs are associated with high costs and are therefore not accessible for every infected patient. Hence, there is a continued need for new antiviral agents for the treatment of viral diseases caused by *Flaviviridae viruses.*

### SUMMARY OF THE INVENTION

In a first aspect, the present invention relates to a pharmaceutical composition comprising a colloidal dispersion of particles of silica having a particle size from 3 nm to 100 nm to which particles silver ions have been adsorbed, for use in a method of treatment of a viral infection.

In another aspect, said antiviral agent and composition are provided in a kit of parts comprising
a) a pharmaceutical formulation including an antiviral agent, optionally in admixture with a pharmaceutically acceptable excipient; and
b) a colloidal dispersion comprising particles of silica having a particle size from 3 nm to 100 nm to which particles silver ions have been adsorbed.

In yet another aspect there is provided a colloidal dispersion comprising particles of silica having a particle size from 3 nm to 100 nm to which particles silver ions have been adsorbed, for use in a method for the treatment of a viral infection, by administration in combination with a further antiviral agent.

In yet another aspect there is provided a pharmaceutical composition as defined herein, further comprising one or more additional antiviral agents.

### BRIEF DESCRIPTION OF THE DRAWINGS

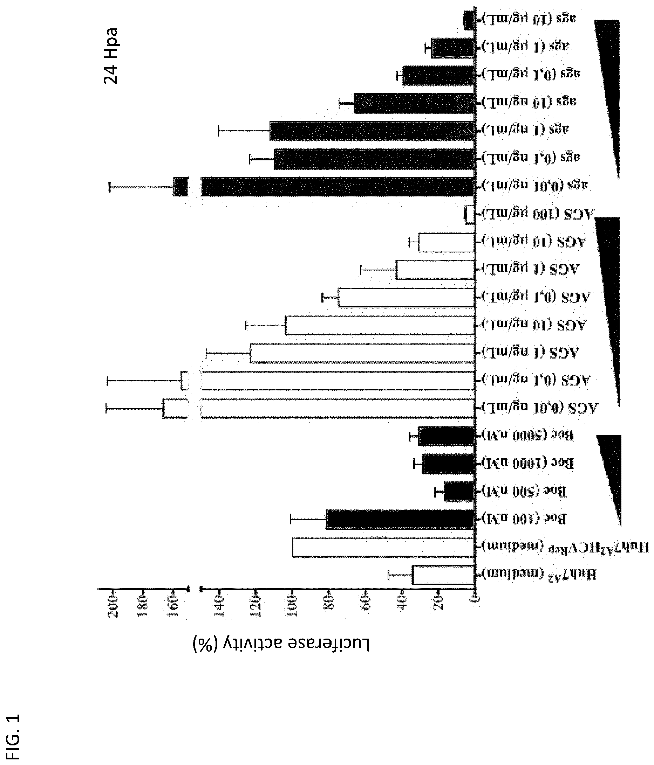

FIGURE 1 shows bar charts representing the luciferase activity measured in Huh7^{A2}HCV^{Rep} cells in the presence or absence of inhibitors at 24 hours post-addition of Boceprevir, AGSOL 1000-5-5 and 100-5-5, respectively. The luciferase activity is represented as percent relative to the positive control (Huh7^{A2}HCV^{Rep} (without inhibitor)). Abbreviations: Boc: Boceprevir; AGS: AGSOL 1000-5-5; ags: AGSOL 100-5-5.

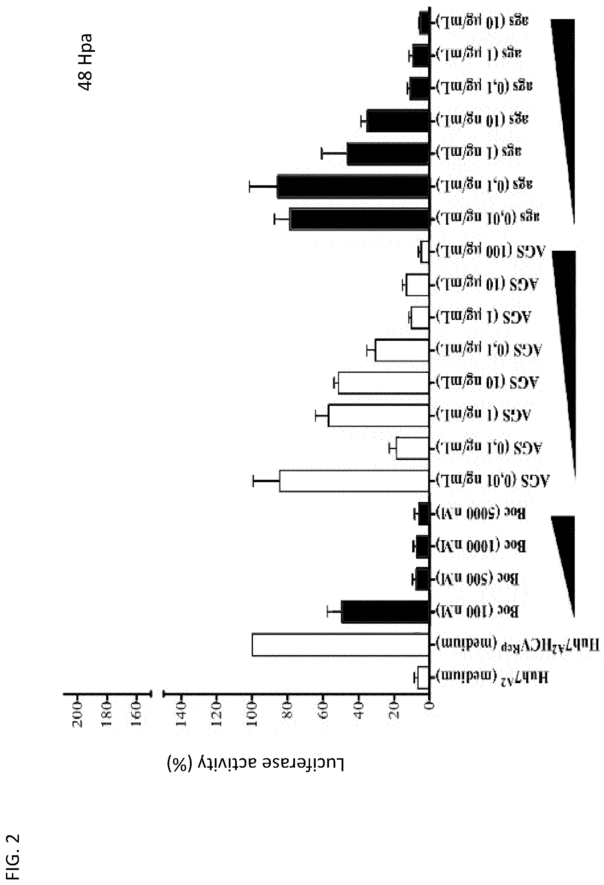

FIGURE 2 shows bar charts representing the luciferase activity measured in Huh7^{A2}HCV^{Rep} cells in the presence or absence of inhibitors at 48 hours post-addition of Boceprevir, AGSOL 1000-5-5 and 100-5-5, respectively. The luciferase activity is represented as percent relative to the positive control (Huh7^{A2}HCV^{Rep} (without inhibitor)). Abbreviations: Boc: Boceprevir; AGS: AGSOL 1000-5-5; ags: AGSOL 100-5-5.

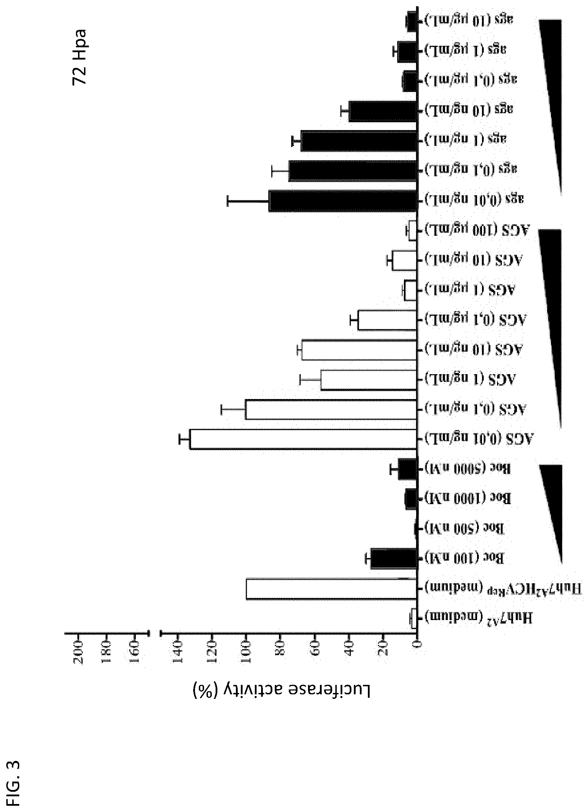

FIGURE 3 shows bar charts representing the luciferase activity measured in Huh7^{A2}HCV^{Rep} cells in the presence or absence of inhibitors at 72 hours post-addition of Boceprevir, AGSOL 1000-5-5 and 100-5-5, respectively. The luciferase activity is represented as percent relative to the positive control (Huh7^{A2}HCV^{Rep} (without inhibitor)). Abbreviations: Boc: Boceprevir; AGS: AGSOL 1000-5-5; ags: AGSOL 100-5-5.

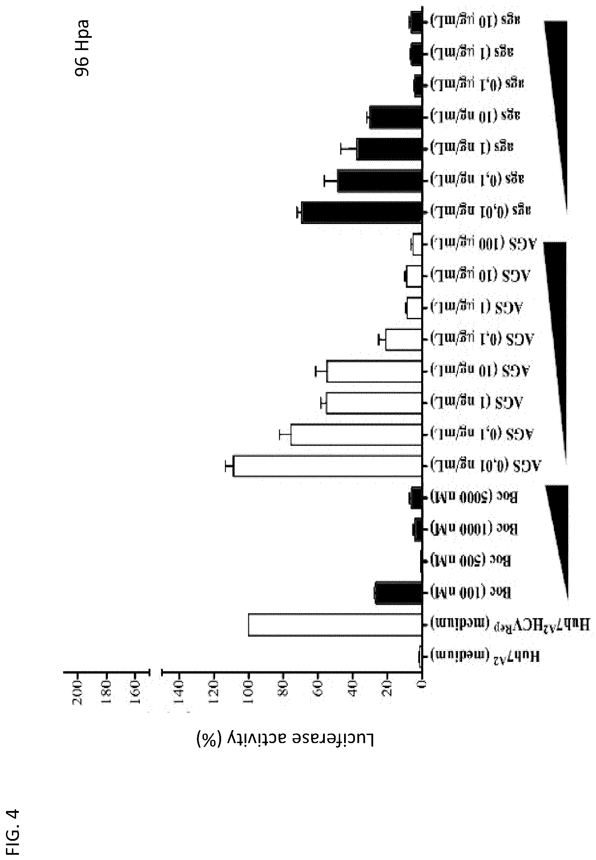

FIGURE 4 shows bar charts representing the luciferase activity measured in Huh7⁴²HCV^{Rep} cells in the presence or absence of inhibitors at 96 hours post-addition of Boceprevir, AGSOL 1000-5-5 and 100-5-5, respectively. The luciferase activity is represented as percent relative to the positive control (Huh7^{A2}HCV^{Rep} (without inhibitor)). Abbreviations: Boc: Boceprevir; AGS: AGSOL 1000-5-5; ags: AGSOL 100-5-5.

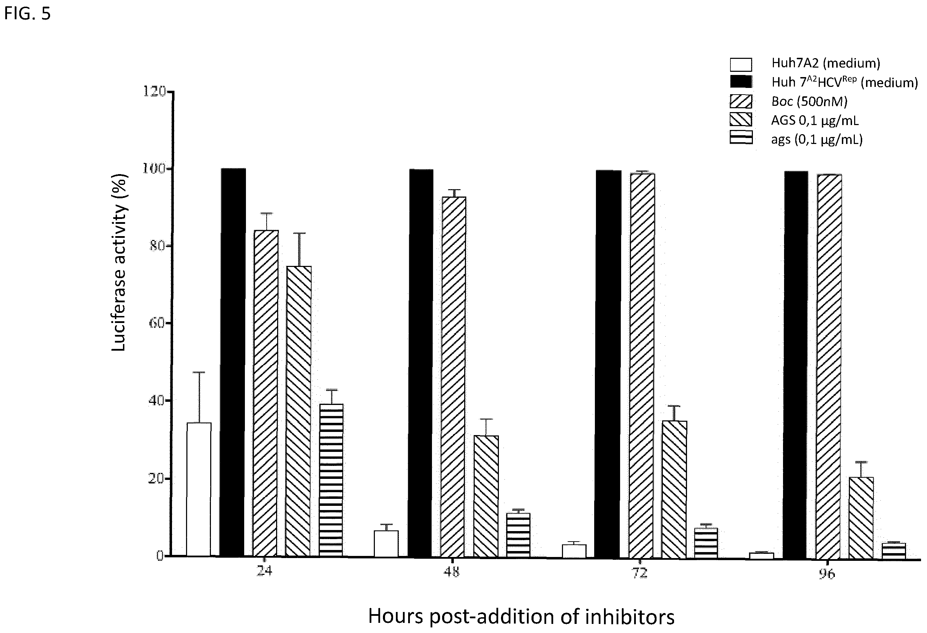

FIGURE 5 is a bar chart showing the luciferase activity measured in Huh7^{A2}HCV^{Rep} cells at 24, 48, 72 and 96 hours post-addition of 500 nM of Boceprevir, 0.1 µg/mL of AGSOL 1000-5-5, and 0.1 µg/mL of AGSOL 100-5-5, respectively. Abbreviations: Boc: Boceprevir; AGS: AGSOL 1000-5-5; ags: AGSOL 100-5-5.

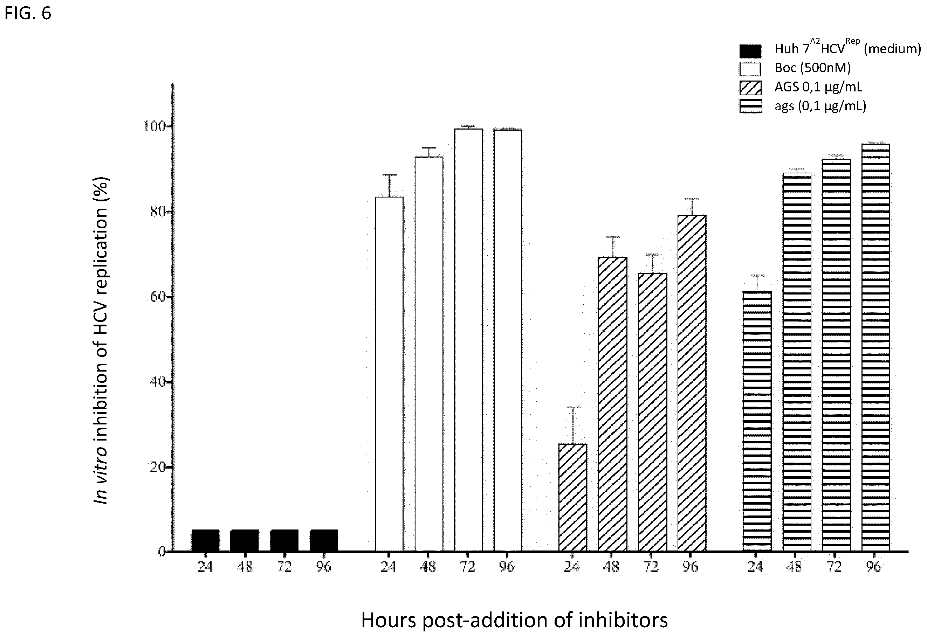

FIGURE 6 is a bar chart showing the inhibition of the HCV *in vitro* replication in Huh7^{A2}HCV^{Rep} cells at 24, 48, 72 and 96 hours post-addition of 500 nM of Boceprevir, 0.1 µg/mL of AGSOL 1000-5-5, and 0.1 µg/mL of AGSOL 100-5-5, respectively. Abbreviations: Boc: Boceprevir; AGS: AGSOL 1000-5-5; ags: AGSOL 100-5-5.

### DETAILED DESCRIPTION OF THE INVENTION

A convenient source of the silica particles used in the present invention are commercial silica sols. Such sols are aqueous dispersions of silica particles and the particles are uniform spheres of silica, which have no internal surface area or detectable crystallinity. They are usually dispersed in an alkaline medium, which reacts with the silica surface to produce a negative charge. Because of the negative charge, the particles repel one another resulting in a stable product.

The solids content of commercial silica sols depends on the particle size and varies from less than 10 % per weight silica for the smallest particles, 3 nm, to about 50 % per weight silica for larger particles, > 20 nm. The surface of the particles in aqueous silica sols is covered with surface hydroxyl groups, silanol groups. The particle size of commercial silica sols is typically in the range of 3-100 nm. The specific surface area (e.g. by SEARS's titration or BET) is generally of 25-1000 m²/g, such as 30-800 m²/g, more preferably 100-600 m²/g, even more preferably 200-600 m²/g, and most preferably 200-550 m²/g.

Stabilization of commercial silica sols is accomplished by adjusting the pH of the sol to between 8.0 and 10.0 by addition of alkali, usually a solution of sodium hydroxide. The sols also contain small amounts of other electrolytes such as sodium chloride and sodium sulfate. The stability of highly concentrated silica sols is very sensitive to the presence of electrolytes. The electrolyte concentration can be reduced to a minimum by using ion exchange resins.

In some commercial products, the particle surface is modified with aluminosilicate ions so as to provide the surface with a fixed, pH-independent negative charge that will make the products more stable towards gelling than the sols from which they were prepared. Trivalent aluminium atoms have been substituted for part of the tetravalent silicon atoms in the surface of the particles, creating a fixed negative charge which is independent of pH. The surface coverage of Al is much less than that corresponding to a Langmuir monolayer.

An example of commercial silica sols useful in the context of the present invention, are the Bindzil^{®} series sold by Akzo Nobel AB.

A colloidal dispersion of as described herein, named "AgSol" comprise silica particles having a particle size from 3 nm to 100 nm carrying silver ions on at least part of the surface of said particles. Such colloidal dispersions may be prepared by contacting colloidal silica with a solution of silver nitrate (AgNO₃) in water. Notably, however, most soluble silver salts can be used to prepare the dispersion presented herein. The colloidal sols as used herein may be prepared by methods as described in WO 2011/037523 A1.

The silver ions are attached ("adsorbed") to the surface, and the adsorption may be by electrostatic or ionic bonding or any other type of bonding, e.g. partly covalent bonding. The adsorption of metal ions on the surface of silica particles may be monitored by measuring the Zeta potential of the colloidal sol.

The particles of the present invention have a particle size from 3 nm to 100 nm, such as from 3 to 75 nm, 3 to 50 nm, 3 to 25 nm, 3 to 20 nm, 3 to 15 nm, 3 to 10 nm, 3, to 8 nm, or 3 to 5 nm.

The charge of metal ions in solution, usually aqueous solutions, is normally positive. This is the case for silver, which usually, but not always, forms monovalent cations in aqueous solutions. To achieve strong adsorption of metal ions on the surface of nano-sized carrier particles the electrical charge of the latter should be high but of opposite charge to that of the metal ions. The charge on the particles in colloidal silica or the particles of silica in an aqueous environment increases exponentially with pH and is almost 0.5 units of negative charge per nm² particle surface at pH of about 10 and at very low, 10⁻⁴ normal, electrolyte concentrations. Colloidal silica has a local stability maximum at the point of zero charge, which occurs at about pH 2.0. The stability of a silica sol first decreases with pH and reaches a minimum around pH 6, after which the sol enters a region of high stability between pH 8 and pH 10.5.

The surface charge of silica, and of many other metal oxides as well, can be altered by modifying the surface in different ways. In one method, when the particle surface of silica sols is modified with aluminosilicate ions (e.g. by treating sols with sodium aluminate solution) to create aluminosilicate sites on the particle surface, the surface will have a fixed, pH-independent negative charge that will make the sol more stable towards gelling by the presence of electrolytes and at low pH, for instance pH 4 to pH 5, than the sols from which they were prepared. Therefore, the particles of silica described herein may have aluminosilicate sites at the particle surface.

A convenient way to introduce aluminosilicate sites on the surface of colloidal silica particles is to use weak acid cation resin to remove sodium ions from the silica sol - sodium aluminate system and thus bring about reaction of the aluminate ions with the surface of the silica particle. In this system, pH will usually not fall below pH 5 even if an excess of weak acid cation exchange resin is used.

A calculated amount of sodium aluminate solution to give the desired number of aluminosilicate sites per nm² particle surface is simply added to the slurry of colloidal silica and resin. The creation of aluminosilicate sites on the surface of silica is also well described in the literature, (e.g. in ller, The Chemistry of Silica, 1979, pp. 407-409). Such descriptions also indicate that it is difficult to introduce much more than about 2 aluminosilicate sites per nm² silica surface, for example. The concentration of aluminosilicate sites on the surface of preferred AgSol particles comprised herein falls in the range from about 0.20-2.0 site per nm², e.g. 0.30-1.50, or 0.3-1.25, or 0.4-1.0 site per nm², e.g. 0.4-0.8 site per nm².

The aluminosilicate sites carry a negative charge, which must be neutralized by counter ions, most often Na⁺ ions. Modification of the silica surface with sodium aluminate converts the surface to a cation exchanger.

Although adsorption of metal cations on aluminosilicate-modified silica sols can be carried out over a wide pH range it is preferable to carry out the adsorption in the pH range where silica sols are most stable; that is the alkaline range, for instance in the pH range from about 8 to about 10.5. However, adsorption of metal cations on aluminosilicate-modified silica sols can be carried out over a wide pH range, e.g. from about pH of about 3 to a pH of about 12, e.g. from a pH of about 4 to a pH of about 11.5, or a pH of about 5 to a pH of about 11, e.g. a pH of about 6 to a pH of about 10.5.

The pH can be controlled at different steps of the process for making the composite sols. Thus, the silica sol can be added to the transition metal salt solution and the pH then adjusted to between 10 and 11 by adding a strong base, e.g. 1 M NaOH-solution, to the metal containing silica sol. Alternatively, alkali can be added to the silica sol before said sol is added to the metal salt solution or before the metal salt solution is added to said sol.

The rate at which silver salt solution can be added to the silica sol without destabilizing the sol depends on the conditions being used in the preparation. The rate of addition can be fast as long as the increments of added salt are virtually instantaneously dispersed throughout the sol and there rapidly adsorbed onto the silica particles. In many small scale preparations it is actually possible to inject 0.1 m AgNO₃ solution into magnetically stirred silica sols in very short times, for instance 10-15 seconds, without destabilizing the sols. However, in most of the small scale laboratory preparations, for instance preparations of sols containing about 500 ppm of metal, longer addition times of 0.1 molar silver salt solutions, typically 2-3 minutes are suitably used so as to be on the safe side in terms of having good stability towards gelling or aggregation. Sols with higher silver contents may require longer times of addition. Thus, a sol containing 1500 ppm silver may require a time of addition of 0.1 molar silver solutions of about 12 minutes. Similar time scales will apply to larger scale preparations provided that agitation or stirring is as efficient as in the small scale preparations.

A sol of a given concentration of silver can be prepared in different ways. The reactants and products used in the various preparations and methods fall in the domain of colloids and colloid chemistry and due care has to be taken concerning concentration of reactants and products, maintaining a high electrical charge on colloidal particles, using water of good quality, preferably deionized water, observing proper rate of addition and order of addition of the components, working in conservative but realistic temperature ranges and providing sufficient agitation and stirring so as to maintain stability towards gelling or aggregation of reactants and products. Selecting and optimizing conditions of the before mentioned type are considered to be within the capacity of the person of ordinary skill in the art.

In one method, a certain amount of silver nitrate solution is added to a silica sol with specified values of particle size and concentration of silica. In another method, the same amount of silver nitrate solution is added to a sol of the same particle size but higher, for instance four times higher, concentration of silica. The overall concentration of silver is the same in the two sols but the concentration of silver on the particle surface of the former sol is higher - four times higher - than that of the latter sol. Thus, a colloidal dispersion with a given, overall concentration of silver and a given particle size may be obtained by combining high concentration of particles, that is a high concentration of silica, with a low concentration of silver on the particle surfaces or by combining high surface concentration of silver with low silica concentration.

The concentrations of silica of the sols used herein may vary from less than 0.1 % SiO₂ to 50 % SiO₂, preferably 0.5-30 % SiO₂, or 1-25 % SiO₂, or 1-10 % SiO₂, e.g. 2-10 % SiO₂ by weight, the remaining part (adding up to 100 %) comprising e.g. silver ions and water.

An example of a colloidal dispersion presented herein comprises particles of silica to which silver ions have been adsorbed, wherein the silver ions are present in an amount of 0.05 ppm to 10 000 ppm by weight of the colloidal dispersion, such as 0.05 to 500 ppm, 0.05 to 200 ppm, 0.05 to 100 ppm, 0.05 to 50 ppm, 0.05 to 10 ppm, or 0.05 to 5 ppm by weight of the colloidal dispersion. The silver ions may also be present in an amount of 0.1 to 500 ppm, 0.1 to 200 ppm, 0.1 to 100 ppm, 0.1 to 50 ppm, 0.1 to 10 ppm or 0.1 to 5 ppm by weight of the colloidal dispersion.

The silver ions may also be present in an amount of 0.2 to 500 ppm, 0.2 to 200 ppm, 0.2 to 100 ppm, 0.2 to 50 ppm, 0.2 to 10 ppm, or 0.2 to 5 ppm by weight of the colloidal dispersion, or 0.3 to 500 ppm, 0.3 to 200 ppm, 0.3 to 100 ppm, 0.3 to 50 ppm, 0.3 to 10 ppm or 0.3 to 5 ppm by weight of the colloidal dispersion, or of 0.4 to 500 ppm, 0.4 to 200 ppm, 0.4 to 100 ppm, 0.4 to 50 ppm, 0.4 to 10 ppm or 0.4 to 5 ppm by weight of the colloidal dispersion.

The silver ions may also be present in an amount of 0.5 to 500 ppm, 0.5 to 200 ppm, 0.5 to 100 ppm, 0.5 to 50 ppm, 0.5 to 10 ppm or 0.5 to 5 ppm by weight of the colloidal dispersion, or 1 to 5000 ppm, 1 to 3000 ppm, 1 to 2000 ppm, 1 to 500 ppm, 1 to 200 ppm, 1 to 100 ppm, 1 to 50 ppm, 1 to 10 ppm or 1 to 5 ppm by weight of the colloidal dispersion or 5 ppm to 10 000 ppm by weight of the colloidal dispersion, such as 5 ppm to 10 000 ppm, 5 ppm to 5 000 ppm or 100 ppm to 5 000 ppm by weight of the colloidal dispersion.

Knowing the concentration of silver in the sol, the atomic weight of silver, the specific surface area of the silica particles and concentration of silica in weight percent, the surface concentration, Cₛ, of silver atoms (ions) per nm² of SiO₂ particle surface, can be calculated according to equation (1): ${C}_{s} = 60 {M}_{1} / \left({M}_{met}AK\right) = \left(60/AK\right) \left({M}_{1}/{M}_{met}\right)$ wherein
M₁ is the concentration of silver in the sol, in ppm,
Mₘₑₜ is the atomic weight of the silver, in g,
A is the specific surface area of the sol particles, in m²/g, and
K is the concentration of silica in weight percent

The concentration of silver ions on the surface of a preferred particle comprising a material used herein falls in the range from about 0.0005 (0.005) to more than 5 silver ions per nm², or from about 0.01 silver ion per nm² to more than 5 silver ions per nm², or from about 0.01 silver ions per nm² to about 2 silver ions per nm², e.g. about 0.01 to about 1 silver ion per nm², or about 0.05 to about 1 silver ion per nm², more preferably about 0.1 to about 0.8 silver ion per nm². Preferably, the concentration is 0.20 - 2.0, more preferably 0.50- 1.50, and even more preferably 0.70 - 1.25 ions (species) per nm².

In the case of silica particles having aluminosilicate sites at the surface, typically, one silver ion adsorbs on one charged Al-Si-site but not all Al-Si sites may have adsorbed silver species adsorbed on them. The ratio by number between silver ions and Al-Si sites may vary within 0.01 - 1.0, but is preferably between 0.05 - 0.8, e.g. between 0.1 and 0.6.

The load of silver ions vs. silica particles may be expressed either as number of silver cations per unit of surface area of the silica particles. This is the "specific silver load" or surface concentration of the silver cation cₛ.

The load of silver ion vs. silica particles in the composite sol may also be expressed as the number of silver ions nₘ for each silica particle. However, for very low silver loads, it may be more meaningful to express the relationship between the number of silver ions and number of particles in the silica sol as the inverse of the number of silver ions for each silica particle, i.e. ${n}_{m}^{- 1}$.

The relationship between ${n}_{m}^{- 1}$ and cₛ is given by the equation (2): ${n}_{m}^{- 1} = \frac{1}{{n}_{m}} ∗ \frac{{A}_{p}}{{A}_{p}} = \frac{1}{{c}_{s} ∗ {A}_{p}}$ wherein
nₘ is the number of silver ions per silica particle in the composite sol,
Ap is the surface area of one silica particle in the composite sol, and
cₛ is the surface concentration of silver ions at the surface of the silica particle.

Equation (2) shows that ${n}_{m}^{- 1}$ is inversely proportional to the surface area Aₚ of the particle and the surface concentration cₛ of silver ions at the surface of the silica particles.

For cₛ = 0.0005 ions/nm², Table 1 illustrates how ${n}_{m}^{- 1}$, viz. the number of silica particles per ion, varies as a function of the silica particle diameter.

In Table 1, the number of silica particles per silver ion as a function of particle diameter in a composite sol, cₛ = 0.0005 ions/nm² is indicated.

**Table 1**

| Particle diameter (nm) | number of particles per ion |
|---|---|
| 5 | 25 |
| 7 | 13 |
| 12 | 4.4 |
| 22 | 1.3 |

As may be seen from Table 1, at cₛ = 0.0005 ions/nm² and a particle diameter of 5 nm, 4 out of 100 silica particles in the composite sol of the invention carry a silver cation, viz. there are 25 particles present for each silver ion in the composite sol.

In other words, the number ratio between silver ions and silica particles in the composite sol of the present invention may vary from high values, where more than one silver ion is present for each silica nanoparticle, e.g. more than 10 silver ions are present for each silica nanoparticle, to low values, where more than one silica nanoparticle is present for each silver ion, e.g. more than 10 silica nanoparticles are present for each silver ion. In some embodiments, the particles comprise from 2 to 25 silver ions per silica particle.

The term "stable" used herein may in some aspects means that the product should be stable toward gelling, implying that the relative viscosity should not increase more than 100 % (e.g. from 5 to 10 mPas) under a period of about two months. The term may also mean stability toward precipitation; i.e. there is no substantial precipitation of solid content, characterised by that no more than 20 % of the solid material has precipitated and settled as a sludge at the bottom, if stored under normal (e.g. ambient or optionally protected from light) conditions, for a period of two months.

As mentioned previously herein, although silica sols are stable over a wide pH range it is preferable to prepare the colloidal dispersions of the invention in the pH region of 8-12, more particularly 9-11, where silica sols are most stable.

In some embodiments, the present invention relates to a pharmaceutical composition comprising a colloidal dispersion of particles of silica having a particle size from 3 nm to 100 nm to which particles silver ions have been adsorbed, for use in a method of treatment of a viral infection. The colloidal dispersion (or sol) is as described herein. For example, the silica particles may have a size ranging from 3 to 100 nm, e.g. 3 to 75 nm, 3 to 50 nm, 3 nm to 25 nm, 3 nm to 10 nm, or 3 nm to 5 nm. In some embodiment, the silica particles have a particle size of 3 to 25 nm. Silver ions may be present in an amount of 0.05 ppm to 50 ppm by weight of the dispersion such as 0.05 ppm to 5 ppm by weight of the colloidal dispersion, or as otherwise defined herein. In some embodiments, the silver ions are present in an amount of 0.5 ppm to 10 ppm by weight of the dispersion.

In some embodiments, the composition does not comprise any further antiviral agent. In some other embodiments, the composition comprises at least one further antiviral agent.

The expression "a colloidal dispersion of particles of silica having a particle size from 3 nm to 100 nm to which particles silver ions have been adsorbed" may also sometimes be referred to herein as a "colloidal dispersion", a "colloidal dispersion containing silver", or a "colloidal dispersion containing silver ions" or the like. Sometimes it is also simply referred to as "silica sol", "composite sol" or a "sol" herein.

There is also provided herein a kit of parts comprising a pharmaceutical composition as defined herein, and a package insert comprising instructions for using the composition, and optionally including a further antiviral agent, e.g. selected from any of the antiviral agents mentioned herein.

In a further aspect, there is provided a pharmaceutical composition comprising a colloidal dispersion of particles of silica having a particle size from 3 nm to 100 nm to which particles silver ions have been adsorbed, for use in a method for the treatment of a viral infection by administering said composition to a subject in need thereof, e.g. a human or an animal, in combination with a further antiviral agent. The composition of the invention and the further antiviral agent may be administered simultaneously, sequentially, or separately.

The colloidal dispersion as defined herein and a further antiviral agent may be administered separately, as separate compositions, or they may be present in the same pharmaceutical composition.

The therapeutically effective, or the pharmaceutically effective amount of the further antiviral agent in combination with the colloidal dispersion as presented herein depends e.g. on the selected antiviral agent, the species of mammal to be treated, e.g. a human or an animal, the body weight, the age, the individual condition, individual pharmacokinetic data, the disease to be treated and the mode of administration.

Any use of a colloidal dispersion as described herein includes uses at least in human and veterinary medicine applications.

Notably, when a treatment of a viral infection is mentioned herein, such a treatment includes e.g. the treatment of a viral infection of the skin or of any mucosal surface.

The administration of the colloidal dispersion and optionally an antiviral agent, or the pharmaceutical composition as described herein, may be effected by local or systemic administration. Systemic administration may e.g. be effected via the oral, parenteral, rectal or pulmonary route. Local administration may e.g. be effected via the topical, oral, rectal or pulmonary route. Particularly, local administration to the intestines and the rectal area is envisaged herein. Furthermore, topical administration to the skin is also particularly envisaged.

Said administration comprises administering a therapeutically effective amount of the colloidal dispersion and optionally a further antiviral agent, or the pharmaceutical composition optionally in association with a (i.e. one or more) pharmaceutically acceptable excipient, e.g. a pharmaceutically acceptable carrier.

For enteral, e.g. oral, administration, the pharmaceutical composition may be formulated in a wide variety of dosage forms. The pharmaceutically acceptable carriers may be solid, semisolid or liquid. Solid form preparations include powders, tablets, pills, lozenges, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The formulation may comprise an encapsulating material as carrier, providing a capsule in which the colloidal dispersion and an antiviral agent, with or without carriers, is surrounded by a carrier, which is in association with it.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water with viscous material, such as natural or synthetic gums, resins, methylcellulose, sodium carboxymethylcellulose, and other well-known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The colloidal dispersion and an antiviral agent, or the pharmaceutical composition, may also be administered parenterally, e.g. by, injection or infusion, e.g. by intravenous, intraarterial, intraosseous, intramuscular, intracerebral, intracerebroventricular, intrasynovial, intrasternal, intrathecal, intralesional, intracranial, intratumoral, intracutaneous and subcutaneous injection or infusion.

Thus, for parenteral administration, the pharmaceutical compositions of the invention may be in the form of a sterile injectable or infusible preparation, for example, as a sterile aqueous or oleaginous suspension. This suspension may be formulated according to techniques known in the art using suitable dispersing or wetting agents (e.g., Tween 80), and suspending agents. The sterile injectable or infusible preparation may also be a sterile injectable or infusible solution or suspension in a non-toxic parenterally acceptable diluent or solvent. For example, the pharmaceutical composition may be a solution in 1,3-butanediol. Other examples of acceptable vehicles and solvents that may be employed in the compositions of the present invention include, but are not limited to, mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant.

Solutions for parenteral use also may contain suitable stabilizing agents, and if necessary, buffer substances. Suitable stabilizing agents include antioxidizing agents, such as sodium bisulfate, sodium sulfite or ascorbic acid, either alone or combined, citric acid and its salts and sodium EDTA. Parenteral solutions may also contain preservatives, such as benzalkonium chloride, methyl- or propyl-paraben, and chlorobutanol.

For inhalation (pulmonal or nasal administration), suitable pharmaceutical formulations are as particles, aerosols, powders, mists or droplets, e.g. with an average size of about 10 µm in diameter or less. For example, compositions or formulations for inhalation may be prepared as solutions in saline, employing benzyl alcohol or other suitable preservatives, absorption promoters to enhance bioavailability, fluorocarbons, and/or other solubilizing or dispersing agents known in the art.

The pharmaceutical composition of the invention also may be administered topically, to the skin or to a mucous membrane. For topical application, the composition may be e.g. a lotion, a cream, a gel, a paste, a tincture, a transdermal patch, a spray or a gel for transdermal and/or transmucosal delivery. The composition may be in the form of a solution or suspension. The composition may be formulated with a suitable ointment containing the active components suspended or dissolved in a carrier. Carriers for topical administration of the composition include, but are not limited to, mineral oil, liquid petroleum, white petroleum, propylene glycol, polyoxyethylene polyoxypropylene compound, emulsifying wax and water. Alternatively, the pharmaceutical composition may be formulated as a suitable lotion or cream containing the active compound suspended or dissolved in a carrier. Suitable carriers include, but are not limited to, mineral oil, sorbitan monostearate, polysorbate 60, cetyl esters wax, cetaryl alcohol, 2-octyldodecanol, benzyl alcohol and water. The pharmaceutical compositions may also be topically applied to the lower intestinal tract by rectal suppository formulation or in a suitable enema formulation.

Suitable pharmaceutical excipients, e.g. carriers, and methods of preparing pharmaceutical dosage forms are described in Remington's Pharmaceutical Sciences, Mack Publishing Company, a standard reference text in art of drug formulation.

In general, the pharmaceutical composition or the colloidal dispersion and optionally a further antiviral agent will be administered in a therapeutically effective amount by any of the accepted modes of administration for agents that serve similar utilities. Suitable daily dosages typically ranges from 1 to 1500 mg, e.g. 1-500 mg daily, or 1-50 mg daily, but will also depend upon numerous factors such as the severity of the disease to be treated, the age and relative health of the patient, the route and form of administration, and the indication towards which the administration is directed, etc. One of ordinary skill in the art of treating such diseases will be able, without undue experimentation and in reliance upon personal knowledge and the disclosure of this application, to ascertain a therapeutically effective amount of the pharmaceutical composition (or the colloidal dispersion and optionally a further antiviral agent) presented herein for a given disease. It is envisaged that the therapeutically effective dose of an antiviral agent may be reduced when the antiviral agent is administered in combination with the colloidal dispersion presented herein.

In some embodiments, it is envisaged that administration is effected by the topical route, such as in the form of a paste, lotion, cream, spray or an ointment. Administration can also be effected through a patch, or the like, to which a pharmaceutically effective amount of the pharmaceutical composition as described herein (or of the colloidal dispersion described herein and optionally a further antiviral agent) has been absorbed.

In some other embodiments, it may be suitable to administer the pharmaceutical composition as described herein (or the colloidal dispersion described herein and optionally a further antiviral agent) orally, or even more preferably by the pulmonary route (e.g. by inhalation), such as by using a pulmonary drug delivery platform such as a pressurized metered-dose inhaler (pMDI), Dried-Powder Inhaler (DPI), aqueous metered-dose inhaler (MDI)/small volume nebulizer, a Soft Mist Inhaler or the like, or by nebulisation, such as by using a nasal spray of an inhalation device.

Particularly, viral infections of the skin or mucous surface caused e.g. by herpes virus may advantageously be treated via the topical route e.g. by using a paste, cream, lotion or an ointment comprising the pharmaceutical composition or the colloidal dispersion solely or in combination with an antiviral agent as provided herein, or by using a spray making it possible to partly or fully cover the affected area to prevent spread of the viral infection.

The antiviral pharmaceutical composition provided herein is considered to have a broad-spectrum antiviral effect, and therefore to be useful for the treatment of a wide variety of viral diseases. Indeed, as will be shown herein below, an antiviral effect was provided by the colloidal dispersion of the invention, against as different viruses as herpes simplex virus, an enveloped, relatively large double-stranded DNA virus belonging to the *Herpesviridae* family, and hepatit C virus, a small enveloped, positive-sense single-stranded RNA virus of the *Flaviviridae* family.

Consequently, in some advantageous embodiments a broad spectrum antiviral activity is provided by the colloidal dispersion as described herein. In some embodiments, it is contemplated that the pharmaceutical composition provided herein may be useful for the treatment of wide variety of viruses, e.g. selected from DNA viruses such as viruses belonging to *Herpesviridae* family and from RNA viruses, such as viruses belonging to the *Flaviviridae* family.

In some embodiments, the virus is a DNA virus, e.g. an enveloped, double-stranded DNA virus, such as a virus belonging to the *Herpesviridae* family.

In some embodiments, a virus belonging to the *Herpesviridae* family more particularly is a virus belonging to the *Cytomegalovirus, Lymphocryptovirus, Simplexvirus,* or *Varicellovirus* genus, in particular the *Simplexvirus* genus.

In some embodiments a virus belonging to the *Herpesviridae* family, more particularly is selected from *Herpes simplex* viruses 1 and 2, *Human alphaherpesvirus 3* (varicella zoster virus), Epstein-Barr virus, human cytomegalovirus, human herpesvirus 6A and 6B, human herpesvirus 7, and Kaposi's sarcoma-associated herpesvirus.

In some embodiments, a virus as mentioned herein, belonging to the *Herpesviridae* family, more particularly is selected from herpes simplex viruses 1 and 2.

In some further embodiments, the virus is an RNA virus, e.g. an enveloped, single-stranded RNA virus, such as a virus belonging to the *Flaviviridae* family.

In some embodiments, a virus belonging to the *Flaviviridae* family more particularly is a virus belonging to the *Flavivirus* genus (e.g. the Yellow fever virus, West Nile virus, Dengue Fever or Zika virus), the *Hepacivirus* genus (e.g. *Hepacivirus C* or the *Hepacivirus B*), the *Pegivirus* genus, or the *Pestivirus* genus. In some embodiments, the virus is a virus of any of the genuses *Hepacivirus, Simplexvirus, Pegivirus,* and *Pestivirus.*

In some embodiments, a virus belonging to the *Flaviviridae* family more particularly is a virus belonging to the *Flavivirus* genus or the *Hepacivirus* genus, in particular the *Hepacivirus* genus.

In some embodiments, a virus belonging to the *Flaviviridae* family more particularly is *Hepacivirus C.*

In some embodiments, the virus is selected from *Herpes simplex* viruses 1 and 2, varicella zoster virus, Epstein-Barr virus, human cytomegalovirus, human herpesvirus 6A and 6B, human herpesvirus 7, Kaposi's sarcoma-associated herpesvirus, and hepatitis virus C (HCV).

In some further embodiments, the virus is selected from hepatitis virus C (HCV), human herpes virus 1 (HSV-1), and human herpes virus 2 (HSV-2). In some embodiments, the virus is hepatitis virus C (HCV). In some embodiments, the virus is human herpes virus 1 (HSV-1). In some embodiments, the virus is human herpes virus 2 (HSV-2).

The disorder treated according to the present invention is a viral infection or linked to a viral infection, e.g. an infection by a virus as mentioned herein. In some embodiments, the disease is a herpes infection, such as orofacial herpes (herpes labialis). In some other embodiments, the disease is genital herpes.

As will be shown herein below, topical application of the colloidal dispersion as provided herein is effective for the relief of symptoms of orofacial herpes, such as blisters and itching, and therefore an advantageous embodiment of the invention relates to a method for topical treatment of orofacial herpes, and to a pharmaceutical formulation for use in such a method.

Thus, provided herein is a pharmaceutical formulation for treatment of orofacial herpes, e.g. in the form of a colloidal dispersion, or a cream, an ointment, a gel, etc. for applying to and around mouth and lips at a threatening outbreak of orofacial (labial) herpes. However, the formulation of the invention is not limited to orofacial herpes, but to any herpes infection, e.g. herpes infection of the eyes. Thus, also provided herein is a pharmaceutical formulation for treatment of eye herpes, e.g. in the form of a colloidal dispersion, or a cream, a gel, for applying to and around the eyes at a threatening outbreak of eye herpes.

Other viral disorders that may suitably be treated by the pharmaceutical composition (also referred to as formulation) of the invention are an infection by any of the viruses mentioned herein, e.g. hepatitis. In such disorder, pharmaceutical composition of the invention may be administered as an oral formulation, e.g. as a capsule, or as a drinkable dispersion.

In some embodiments, the colloidal dispersion of the invention is used in combination with a further antiviral agent. The term "antiviral agent" may be generally defined as an agent capable of inhibit the development of a virus. In some embodiments, the further antiviral agent is selected from antiviral interferons, non-structural protein 5A (NS5A) inhibitors, protease inhibitors, purine nucleosides, miscellaneous antivirals, and antiviral combinations.

In some embodiments, said additional antiviral agent is an antiviral agent intended for the treatment or prevention of HSV-1, such as acyclovir or a functional equivalent thereof.

In some embodiments, said antiviral agent(s) is an antiviral agent intended for the treatment or prevention of HCV, such as Boceprevir or a functional equivalent thereof.

In some embodiments, the further antiviral agent is selected from antiviral interferons. In some embodiments, the further antiviral agent is selected from non-structural protein 5A (NS5A) inhibitors. In some embodiments, the further antiviral agent is selected from protease inhibitors. In some embodiments, the further antiviral agent is selected from purine nucleosides. In some embodiments, the further antiviral agent is selected from miscellaneous antivirals. In some embodiments, the further antiviral agent is selected from antiviral combinations.

Examples of antiviral interferons are peginterferon alfa-2a and peginterferon alfa-2b.

Daclatasvir is an example of an NS5A inhibitor.

Examples of protease inhibitors are boceprevir, simeprevir, and telaprevir.

Examples of purine nucleosides are ganciclovir, ribavirin, acyclovir, famciclovir, and valacyclovir.

Examples of miscellaneous antivirals are fomivirsen, sofosbuvir, enfuvirtide, foscarnet, letermovir, ibalizumab, and baloxavir marboxil.

Examples of antiviral combinations are combinations consisting of: glecaprevir and pibrentasvir; elbasvir and grazoprevir; ledipasvir and sofosbuvir; sofosbuvir and velpatasvir; dasabuvir, ombitasvir, paritaprevir and ritonavir; ombitasvir, paritaprevir, and ritonavir; dasabuvir, ombitasvir, paritaprevir and ritonavir; or sofosbuvir, velpatasvir, and voxilaprevir.

The invention will now be further illustrated and exemplified by the following experimental section, but is not intended to be limited thereto.

### EXPERIMENTAL SECTION

### EXAMPLE 1

### ANTIVIRAL ACTIVITY AGAINST HEPATITIS C VIRUS

### MATERIALS AND METHODS

### Cell lines

A hepatoma cell line (Huh7-Lunet) designated Lunet-HlaA2-luc/neoET (Huh7^{A2}HCV^{Rep}) and Lunet-HlaA2 (Huh7^{A2}) were kindly provided by Dr. Volker Lohmann (UniversitätsKlinikum Heidelberg, Molekulare Virologie). The Lunet-HlaA2-luc/neoET expressed ectopically HLA-A2 and a selectable HCV subgenomic RNA replicon of genotype 1b harboring replicationenhancing mutations in NS3 and NS4 (Con1-ET). The replicon also expresses the selectable marker neomycin phosphotransferase, which inactivates the cytotoxic drug G418. The expression of non-structural proteins was driven by the encephalomyocarditis virus IRES (Robinson, M., Yang, H., Peng B. et al. 2010. Antimicrobial Agents and Chemotherapy. 54(8):3099-3106; Pasetto, A., Frelin, L. et al. 2012. J immunol. 189:4510-4519). The replicon cells expressed the firefly luciferase gene fused to the selectable marker by ubiquitin. As control, the parenteral cell line, designated Lunet-HlaA2, was used.

### Cell culture

Both cell lines were grown in DMEM AQmedia^{™}-medium, supplemented with 10% foetal bovine serum (FBS), 100 U.ml⁻¹ penicillin, 100 µg.ml⁻¹ streptomycin, and 1 mM nonessential amino acids. The Lunet-HlaA2-luc/neoET (Huh7^{A2}HCV^{Rep}) cells were grown with selection of puromycin (1 µg/mL) and G418 (0.5 mg/mL) disulfate salt solution. The Lunet-HlaA2 (Huh7^{A2}) cells were grown with selection of 3 µg/ml of blasticidin S hydrochloride (Gibco, New York, NY). Cell lines were grown in T75 flasks (Becton Dickinson and Company, Franklin Lakes, NJ) containing 25 ml of complete medium, in a humidified incubator at 37°C and with 5% CO₂. Except for blasticidin, all medium and supplements were purchased from Sigma-Aldrich (Saint-Louis, MO).

### Antiviral compounds

Boceprevir / SCH 503034 (Victrelis, MSD), an approved DAA for treatment of chronic HCV infection was included in these studies as a positive control. Boceprevir is a ketoamide HCV NS3 protease inhibitor, forming a reversible and covalent bond to the NS3 protease active site The antiviral potency of Boceprevir has already been shown in *in vitro* and *in vivo* studies (SPRINT and RESPOND studies) with a 50% effective concentration (EC₅₀) = 200nM and Ki = 14 nM [10-11]. Thus, Boceprevir was used as a positive control to assess the efficiency of colloidal dispersions provided herein. The antiviral activity was evaluated using the Lunet-HlaA2-luc/neoET (Huh7^{A2}HCV^{Rep}) cells. The tested dispersions were AGSOL 1000-5-5 and 100-5-5, containing silica nanoparticles having a diameter of 5 nm coated with silver ions. AGSOL 1000-5-5 has 20 silver ions on each particle (stock concentration 1000 µg/mL) and AGSOL 100-5-5 has 2 silver ions on each particle (stock concentration 100 µg/mL).

### Cellular growth kinetics

The growth potential of Huh7^{A2}HCV^{Rep} and Huh7^{A2} was assessed by seeding 100 000 or 200 000 cells per well (12-well plate) or petri-dish (35 mm x 10 mm) in 1 mL of complete AQmedia. Cells were incubated (37°C, 5% CO₂) for 1, 3, 5 and 7 days post-seeding. After 1, 3, 5 and 7 days, the medium was removed, cells washed with PBS, thereafter detached using 0.5 mL of trypsin/EDTA (Gibco, New York, NY), and incubated for 4 minutes. Thereafter, 1 mL of medium was added and cells harvested, centrifuged at 1500 rpm for 5 minutes. Supernatant was discarded and the pellet was suspended in 200 µL of complete medium. Cells were counted using a Neubauer chamber (10 µL of cells were taken and mixed with 90 µL of trypan-blue (Sigma-Aldrich, Saint-Louis, MO)). The cellular growth of Huh7^{A2}HCV^{Rep} cell line was represented as the cell count per day post-seeding, and as the percentage of confluence determined by microscopic observation every two-days post-seeding.

### Antiviral assays

Cells were seeded in flat bottom 96-well plates (Nuncleon^{™} Δ Surface, Denmark) at a density of 5 000 cells/well in 100 µL of culture medium without antibiotics (G418, puromycin and blasticidin). Boceprevir, diluted in dimethyl sulfoxide (DMSO) hybrid-max, purchased from Sigma-Aldrich (Saint-Louis, MO), was added at several concentrations: 100 nM, 500 nM, 1000 nM and 5000 nM. The final concentration of DMSO never exceeded 0.2 %. AGSOL 1000-5-5, diluted in culture medium, was added at several concentrations: 0.01 ng/mL, 0.1 ng/mL, 1 ng/mL, 10 ng/mL, 0.1 µg/mL, 1 µg/mL, 10 µg/mL and 100 µg/mL. AGSOL 100-5-5 was added as described above, excluding the concentration 100 µg/mL. Medium without added cells and cells without any inhibitors constituted negative controls. Each concentration of drug was tested in 5 unique wells and the same protocol was carried out for Huh7^{A2}HCV^{Rep} and Huh7^{A2} cell lines. Inhibitors were refreshed daily. The experimental assays were performed three times with similar results.

### MTT tetrazolium assay - proliferation assay

To assess cellular toxicity of the antiviral compounds (Boceprevir and AGSOL), use was made of the *in vitro* toxicology assay kit MTT (Stock No. TOX-1, Sigma-Aldrich, Saint-Louis, MO). The 3-[4,5-dimethyltiazol-2-yl]-2,5-diphenyl tetrazolium bromide is cleaved by mitochondrial dehydrogenases of viable cells, leading to purple formazan in the form of insoluble crystals in aqueous solutions but dissolved in acidified isopropanol. The resulting purple solution is spectrophotometrically measured. A decrease in cell number, due to inhibitors' toxicity, is proportional to a decrease of formazan crystal formation.

At time-points 24, 48, 72 and 96 hours post-addition of inhibitors, culture plates were removed from incubator into laminar flow hood. An amount equal to 10 % of the culture medium volume of reconstituted MTT [Catalog No. M-5655] was added in each well. Plates were returned to incubator for 3 hours. After the incubation period, the resulting formazan crystals were dissolved by adding an amount of MTT Solubilisation Solution [Catalog No. M-8910] equal to the original culture medium volume. Plates were gently mixed in a gyratory shaker to enhance dissolution and spectrophotometric measurement absorbance was achieved at a wavelength of 570 nm. The background absorbance of multi-well plates was measured at 690nm and was subtracted from the 570 nm values. Results were presented as the percentage of viability per condition (inhibitor/concentration) and the absorbance (A₅₇₀ₙₘ - A₆₉₀ₙₘ) per time-point for each inhibitor and per cell line.

### Luciferase activity assay

To assess the antiviral activity of Boceprevir, AGSOL 1000-5-5 and 100-5-5, the ONE-Glo^{™} Luciferase Assay System (Cat.# E6120) purchased from Promega (Madison, WI) was used. For determination of the antiviral activity of Boceprevir and AGSOL the Lunet-HlaA2-luc/neoET (Huh7^{A2}HCV^{Rep}) cells were used with constitutive HCV replication and luciferase expression. The Lunet-HlaA2 (Huh7^{A2}) cell line was only used as negative control in this assay. The firefly luciferase gene expressed by the replicon cells catalyses the monooxygenation of beetle luciferin according to the reaction described in the following reaction scheme: 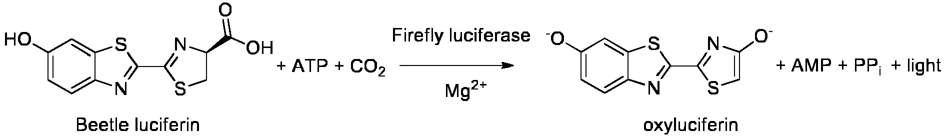 At time-points 24, 48, 72 and 96 hours post-addition of inhibitors, culture plates were removed from incubator into laminar flow hood and an equal volume of Luciferase assay reagent to that of the culture medium in each well was added. Three minutes were allowed for complete cell lysis, and subsequently the luminescence was measured. Absorbance and luminescence were read by Infinite 200 PRO reader (Tecan Group Ltd, Maennedorf, Switzerland). Results were presented as the percentage of luciferase activity per condition (inhibitor/concentration) relative to the positive control (Huh7^{A2}HCV^{Rep} (medium): 100% of luciferase activity) and the *in vitro* inhibition of HCV replication focused on four conditions (cells with medium only, Boceprevir (500 nM), AGSOL 1000-5-5 (0.1 µg/mL) and AGSOL 100-5-5 (0.1 µg/mL)) was represented as the percentage per time-point.

### Statistical analysis

For all experiments the GraphPad Prism 5.0b for Macintosh (GraphPad Software, La Jolla, CA) has been used. For each condition, values represented on the graphs were obtained from the mean of five wells and the error bars were calculated from standard errors of the mean.

### RESULTS

### Growth kinetics of the Huh7^{A2}HCV^{Rep} cell line.

To initially evaluate the *in vitro* growth kinetics of the HCV replicon cell line (Huh7^{A2}HCV^{Rep} one- or two-hundred thousand Huh7^{A2}HCV^{Rep} cells per well (in 12-well plates) or petri dish (35 mm x 10 mm) were seeded. Cells were harvested every two-days for analysis of cell count and percent confluence. This was done to understand the experimental schedule for measurements of cellular proliferation and HCV replication in the following experiments.

The obtained results show that at 1 day post-seeding, 80 000 cells were counted at 70% of confluence per well in the 12-well plate. At 3 days post-seeding, the amount of cells significantly increased to 460 000 cells per well that represented 90% confluence. Onehundred percent of confluence was reached at 5 days post-seeding although a slower cell growth evidence by 510 000 cells per well was observed. At 7 days post-seeding, the number of viable cells was markedly reduced only 159 000 cells per well. Hence, during 2 days 68% were lost compared to previous time point.

For cells growth in petri dishes, the same profile was found as for the cells grown in 12-well plates. A rapid cell growth was observed the first 5 days post-seeding.

Although a marked loss of cells from day 5 and onwards was observed by cell counting, a 100% confluence was observed by microscopic observations. The 100 % cell confluence was found for both cells grown in 12-well plates and petri dishes. This could be due to the fact that the wells or petri dishes were overgrown at around day 5 to 7. Since the growth kinetics of the Huh7^{A2}HCV^{Rep} cells had been previously determined, assays to determine the cell proliferation (e.g. MTT) and the luciferase activity (antiviral activity) could be set up. It was elected to seed 5 000 cells per well in 100 µL of complete medium in flat bottom 96-well plate. For each concentration of inhibitors/ antiviral drugs 5 well replicates (MTT assay) and 4 well replicates for the luciferase activity assay were made.

### Analysis of Huh7^{A2}HCV^{Rep} and Huh7^{A2} cell viability in the presence or absence of antiviral compounds.

At time-points 24, 48, 72 and 96 hours post-addition (hpa) of antiviral drugs Boceprevir, AGSOL 1000-5-5 and AGSOL 100-5-5, the cellular proliferation was determined in the presence or absence of antiviral compounds/inhibitors. The cellular toxicity of the inhibitors was assessed compared to cell grown without inhibitor, whereby Huh7^{A2}HCV^{Rep} and Huh7^{A2} cells grown without any inhibitor were set to 100% cell viability.

No major toxic effects caused by Boceprevir (at concentrations of 100-500 nM) on the Huh7^{A2}HCV^{Rep} and Huh7^{A2} cell lines were observed throughout the experiments. The cellular viability was never lower than 75% compared to cells grown without inhibitor.

The data were consistent with the already existing results obtained in *in vitro* and *in vivo* trials showing the antiviral activity of Boceprevir. Notably, at 24 hours post-addition of inhibitors, an increased cell proliferation of the Huh^{7A} cell line was observed in the presence of Boceprevir, as well as in the presence of AGSOL 1000-5-5 and AGSOL 100-5-5. Both AGSOL 1000-5-5 and AGSOL 100-5-5, at concentrations between 0.001 ng/mL and 0.1 µg/mL were found to have similar profile as Boceprevir with a cellular viability of 70% at lowest. On the other hand, when doses of from 1 µg/mL to 100 µg/mL were used the toxicity was significant with only around 35% cellular viability at the 1 µg/mL dose. AGSOL 1000-5-5 and AGSOL 100-5-5 caused a significant cellular loss/toxicity at doses above 1 µg/mL. Data were similar for both Huh7^{A2}HCV^{Rep} and Huh7^{A2} cell lines. Also by microscopic observations, it was clearly visible that doses of 1 µg/mL to 100 µg/mL caused significant cell death. The cells that were observed had round shape and detached from the surface. Cell counting confirmed a high proportion of cell death.

It should be mentioned that the AGSOL 1000-5-5 and AGSOL 100-5-5 had a pH of 9 to 10. This caused a color change of the cell culture medium at concentrations 10 µg/mL and 100 µg/mL, which interfered with the absorbance measurement. However, no viable cells were present at these concentrations.

When kinetically comparing the absorbance values (A₅₇₀ₙₘ - A₆₉₀ₙₘ) for the different cell lines and conditions, a limited cellular proliferation was found throughout the experiment. Similar results (e.g. limited proliferation) have been previously shown when using a murine hepatoma cell line.

Kinetic analysis of absorbance values at 24, 48, 72 and 96 hours post-addition of inhibitors showed that Boceprevir did not cause any markedly cellular toxicity, indicated by significant drop in the absorbance values. AGSOL 1000-5-5 and AGSOL 100-5-5 concentrations of 1-100 µg/mL were shown to be toxic for the cells, indicated by low absorbance values, whereas concentrations of 0.1 µg/mL or less were not toxic.

### Analysis of the antiviral effects of Boceprevir, AGSOL 1000-5-5 and AGSOL 100-5-5 on HCV replication

The HCV *in vitro* replication was quantified by measuring the reporter gene expression. The Huh7^{A2}HCV^{Rep} cell line expresses both the HCV replicon and the firefly luciferase gene. The measurement of luciferase correlates with the HCV replication activity.

Addition of Boceprevir to the Huh7^{A2}HCV^{Rep} cells was found to have a significant effect on HCV RNA replication. The results show that Boceprevir concentration of 500 nM to 5000 nM efficiently inhibited the luciferase activity at all time-point (FIGURES 1-4). These results were consistent with a 50% inhibition of replication/ luciferase activity at 200 nM Boceprevir, which has been shown in other studies. In scientific literature, most studies used a Boceprevir concentration of 500 nM to obtain an efficient inhibition of HCV *in vitro* replication. The results show that Boceprevir (500 nM) inhibited HCV *in vitro* replication to around 85% at 24 hpa. At 48, 72, and 96 hpa the inhibition was around 90% (FIGURES 5 and 6).

The luciferase activity decreased proportionally with an inverse dose-dependent relationship of AGSOL 1000-5-5 and AGSOL 100-5-5 (FIGURES 1-5). Thus, the obtained results show that the silver nanoparticles have an antiviral effect on HCV replication. However, for the highest concentrations of AGSOL 1000-5-5 and AGSOL 100-5-5 the low luciferase activity was not due to a true inhibition of HCV replication but explained by toxic effects of the nanoparticles. Thus, the results indicate that the 0.1 µg/mL concentration of AGSOL 1000-5-5 as well as AGSOL 100-5-5 was most interesting because it did not cause any toxicity but had antiviral effects on the HCV *in vitro* replication. AGSOL 1000-5-5 inhibited 25 % of the HCV replication at 24 hpa whereas AGSOL 100-5-5 inhibited HCV replication around 65 %. At the end of the experiment, AGSOL 1000-5-5 showed a 75% inhibition and AGSOL 100-5-5 a 90% inhibition of the HCV *in vitro* replication. Compared to AGSOL 1000-5-5, AGSOL 100-5-5 was found to be more efficient in blocking the HCV *in vitro* replication throughout the experiment (FIGURES 5 and 6).

### EXAMPLE 2

### ANTIVIRAL ACTIVITY AGAINST HERPES SIMPLEX 1 VIRUS

The nanoparticles used in Example 2 consisted of a silica core treated with sodium aluminate followed by silver nitrate to incorporate silver ions on the surface of the particles. The particles had a mean diameter of 5 nm, and either about 2 (in "AGSOL100-5-5") or about 20 (in "AGSOL1000-5-5") silver ions per particle (100-5-5 stands for a dispersion containing 100 ppm by weight of silver ions, silica particles having a diameter of 5 nm and 5 % by weight of silica; 1000-5-5 stands for a dispersion containing 1000 ppm by weight of silver ions, silica particles having a diameter of 5 nm and 5 % by weight of silica). In addition, two control sols were provided: BZ15/500 which consists of silica particles and BZ159/500 which consists of silica particles modified with sodium aluminate.

The effect on *Herpes simplex 1* virus was studied in Green Monkey Kidney (GMK) cells provided by the Tissue Culture Laboratory at the Section for Virus Isolation, Clinical Virology Laboratory, Karolinska University Hospital, Huddinge.

### Colloidal dispersions

The particle dispersions were diluted with PBS (0.01M, pH 7.4) to either 10 µg/mL or 1 µg/mL as shown in Table 2 and Table 3

**Table 2**

| AG 100-5-5 (100 µg/mL) stock dispersion | |
|---|---|
| AG 100-5-5, 10 µg/mL (1/10) | 100 µL (AG 100-5-5) |
| | 900 µL PBS |
| AG 100-5-5, 1 µg/mL (1/100) | 100µL (AG 100-5-5 1/10) |
| | 900 µL PBS |

**Table 3**

| AG 1000-5-5 (1000 µg/mL) stock dispersion | |
|---|---|
| AG 1000-5-5, 10 µg/mL (1/100) | 10 µL (AG 1000-5-5) |
| | 990 µL PBS |
| AG 1000-5-5, 1 µg/mL (1/1000) | 100 µL (AG 1000-5-5 1/100) |
| | 900 µL PBS |

### Bindzil 15/500 and Bindzil 159/500 reference material

The concentration of the reference dispersions were 100 µg particles/mL and they were both treated in the same way. A ten-fold (1/10) dilution was first made by taking 100 µL of the stock d and adding it to 900 µL PBS (0.01M, pH 7.4). 1/100 dilution was prepared by taking 100 µL of 1/10 dilution and adding 900 µL PBS.

### Virus isolates

GMK cells were provided by the Tissue Culture unit at the Laboratory for Clinical Virology, KH and obtained as a cell suspension with a concentration of 70,000 cells/mL containing Minimal Essential Medium (MEM) supplemented with 10% inactivated Foetal Bovine Serum (FBS), 30mg/mL benzylpenicillin (Panpharma) and 60mg/mL streptomycin sulphate (Sigma).

Using a 48-well plate, each well was seeded with 35 000 cells/well (0.5mL cell suspension) and incubated for 24 hours in a humidified incubator at 37°C with 5% CO₂, after which time the cells were examined in a microscope and found to have adhered to the well surfaces with approximately 100% confluence.

Two HSV-1 virus isolates were obtained from Cell Culture section Laboratory for Clinical Virology, KH. Each isolate was used to create a dilution series of the original virus isolates. For the first dilution of 10⁻², 20 µL virus isolate was diluted in 2mL PBS (0.01M, pH 7.4). After vortexing, 200 µL of this dilution was added to a tube with 1.8 mL PBS giving a 10⁻³ dilution. The series of dilutions was repeated giving six ten-fold dilutions of each of the virus isolates: 10⁻², 10⁻³, 10⁻⁴, 10⁻⁵, 10⁻⁶, and 10⁻⁷.

Using one 48-well plate per virus isolate dilution series, 0.4 mL of medium was removed from every well. Starting with the most dilute sample, 0.1mL/well of virus dilution was added in sex replicates.

0.5mL Infection Medium (Sigma Medium 199 (Parker) with 30 mg/mL benzylpenicillin (Panpharma), 60 mg/mL streptomycin sulphate (Sigma) and L-Glutamine (2MM), 2% inactivated FBS, 5% Tryptose phosphate Broth (TPB) and 0.1% Dextrose) were added to the negative wells.

The virus was allowed to adsorb to the cells for 2 hours in a humidified incubator at 37°C and 5% CO₂. After adsorption, 0.1 mL of virus inoculate was removed from all infected wells and 0.5 mL of Infection Medium was added. The plates were incubated in a humidified incubator at 37°C and 5% CO₂ for 7 days. Plates were examined in the microscope every day for 7 days for signs of CPE (Cytopathic Effect). In the case of HSV-1, CPE begins as enlargement, rounding and clustering of infected cells. Virus is released from lysed cells and spreads from cell to cell resulting in the eventual destruction of the monolayer. The rate of monolayer destruction is dependent on the virus titer used to infect cells and the absence of CPE indicates the absence of viable virus. CPE is typically present 1-5 days after inoculation with HSV-1. Results for 4 different virus isolates are shown in Table 4.

**Table 4**

| Virus isolate | 2A | 2B | 2C | 3AA |
|---|---|---|---|---|
| TCID₅₀/mL | 4,64E5 | 3,16E4 | 1,00E5 | 3,16E5 |
| FFU/mL | 3,20E5 | 2,18E4 | 6,90E4 | 2,18E5 |
| 10,000 FFU (volume, µL) | 31,25 | 459 | 145 | 46 |

### Assay of antiviral activity

30 glass tubes were seeded with GMK cells acquired from Tissue Culture Laboratory, KH, and were stored in an incubator at 37°C for 4 days. The colloidal dispersions, with or without silver ions (i.e. inventive and reference), were preincubated with 10,000 virus particles. Virus isolate 2A (FFU/mL: 3.20E5) (31 µL) was incubated with 16 µL of AG100-5-5 (1/10 dilution), 16 µL of AG1000 (1/100 dilution), 16 µL of BZ15/500 (1/10 dilution) or 16 µL of BZ159/500 at 37°C for approximately 2 hours. The contents of each tube were then added to the glass tubes giving a final concentration of 0.1 µg/mL of each substance per tube. The tubes were examined on days 1, 2, 3, 4, 5 and 7.

The virus used to infect the positive control and the colloidal dispersions added to the negative control tubes were also incubated at 37°C for approximately 2 hours. The positive control was strongly positive after 24 hours with complete monolayer destruction after 3 days, while the negative control remained negative throughout the seven days. In two of the three replicates containing 0.1 µg/mL AG100-5-5, a complete infection did not occur while in the third tube with this substance, complete monolayer destruction had occurred after three days. All three replicates containing AG1000-5-5 remained negative for 48 hours before slowly developing an infection with varying progress. By the last day, complete infection was established in two of the three tubes while the third tube remained weakly positive with most of the monolayer unaffected by infection. In those tubes containing reference dispersion, complete infection of the monolayer took 48-72 hours. The results are represented in Table 5, where - denotes no infection, + denotes 10-15 CPE per monolayer, ++ denotes infection of approximately half the monolayer and +++ denotes total monolayer infection, and wherein "Pos" stands for positive control and "Neg" stands for negative control (no virus).

**Table 5**

| Sample type | Day 1 | Day 2 | Day 3 | Day 4 | Day 5 | | Day 7 |
|---|---|---|---|---|---|---|---|
| Pos | +++ | +++ | +++ | +++ | +++ | | +++ |
| | +++ | +++ | +++ | +++ | +++ | | +++ |
| | +++ | +++ | +++ | +++ | +++ | | +++ |
| Neg | - | - | - | - | - | | - |
| | - | - | - | - | - | | - |
| | - | - | - | - | - | | - |
| Ag100-5-5 | (+) | + | + | + | + | | + |
| | (+) | + | + | + | + | | + |
| | (+) | ++ | ++ | +++ | +++ | | +++ |
| Ag100-5-5 (Neg) | - | - | - | - | - | | - |
| | - | - | - | - | - | | - |
| | - | - | - | - | - | | - |
| Ag1000-5-5 | - | - | + | + | ++ | | +++ |
| | - | - | + | ++ | +++ | | +++ |
| | - | - | - | (+) | + | | + |
| Ag1000-5-5 (Neg) | - | - | - | - | - | | - |
| | - | - | - | - | - | | - |
| | - | - | - | - | - | | - |
| Bindzil15/500 | ++ | +++ | +++ | +++ | +++ | | +++ |
| | ++ | ++ | +++ | +++ | +++ | | +++ |
| | ++ | ++ | +++ | +++ | +++ | | +++ |
| Bindzil15/500 (Neg) | - | - | - | - | - | | - |
| | - | - | - | - | - | | - |
| | - | - | - | - | - | | - |
| Bindzil159/500 | ++ | +++ | +++ | +++ | +++ | | +++ |
| | + | ++ | +++ | +++ | +++ | | +++ |
| | ++ | +++ | +++ | +++ | +++ | | +++ |
| Bindzil159/500 (Neg) | - | - | - | - | - | | - |
| | - | - | - | - | - | | - |
| | - | - | - | - | - | | - |

### EXAMPLE 3

### IN VIVO TEST OF ANTIVIRAL ACTIVITY AGAINST HERPES LABIALIS (HSV1)

The test subject was a 53-year old Caucasian male patient having a history of *Herpes simplex* 1 outbreaks (orafacial herpes/herpes labialis) since his early twenties. The patient indicated having outbreaks typically triggered by intense sun exposure, and that a typical outbreak would start with a tingling sensation that would develop to painful blisters over a period of a few days. After fully developed blisters, the outbreak would subside over a time period of about 7 to 10 days.

The formulation used in the test was an aqueous dispersion of 100 ppm silver ions and 1.7 % by weight of silica particles having a diameter of 5 nm (AGSOL 100-5-1.7).

The patient applied a few (3-5) drops of the formulation to the affected orofacial area at the very start of an outbreak, i.e. at the as soon as a tingling sensation was felt. As a result, the outbreak was halted and no blisters appeared.

The patient has continued to apply the treatment at each threatening outbreak, presently 5 times, each time with the same good results of halted outbreak and no blistering.

### EXAMPLE 4

### ANTIVIRAL ACTIVITY AGAINST HERPES SIMPLEX 1 VIRUS USING A COMBINATION TREATMENT OF AGSOL AND ACYCLOVIR

Next, a combination treatment comprising AGSOL nanoparticles and acyclovir (a well know anti-viral agent) will be tested to investigate a synergistic anti-viral effect of this combination treatment as compared to using the anti-viral agent alone. The dose requirements of the antiviral agent when used in combination with AGSOL will also be evaluated.

### Materials

The nanoparticles to be used in Example 4 will comprise of a silica core treated with sodium aluminate followed by silver nitrate to incorporate silver ions on the surface of the particles.

The particles will have a mean diameter of 5 nm or 14 nm, and about 2 silver ions per particle ("AGSOL 100-5-1.7" and "AGSOL 100:14:1.7", respectively) (100-5-1.7 stands for a dispersion containing 100 ppm by weight of silver ions, silica particles having a diameter of 5 nm and 1.7 % by weight of silica; 100:14:1.7 stands for a dispersion containing 100 ppm by weight of silver ions, silica particles having a diameter of 14 nm and 1.7 % by weight of silica).

In addition, two control sols will be provided: BZ15/500 which consists of silica particles and BZ159/500 which consists of silica particles modified with sodium aluminate.

Acyclovir will be provided at a final dilution series from 0.1 µg/mL to 10 µg/mL (such as 0.1 µg/mL, 0.5 µg/mL, 1.5 µg/mL or 10µg/mL).

The effect on the combination treatment of AGSOL and acyclovir on *Herpes simplex* 1 virus will be studied in Green Monkey Kidney (GMK) cells provided by the Tissue Culture Laboratory at the Section for Virus Isolation, Clinical Virology Laboratory, Karolinska University Hospital, Huddinge.

### Colloidal dispersions

The particle dispersions will be diluted with PBS (0.01M, pH 7.4) to either 50 µg/mL or 25 µg/mL as shown in Table 2 and Table 3

**Table 2**

| AG 100-5-1.7 (100 µg/mL) stock dispersion | |
|---|---|
| AG 100-5-1.7, 50 µg/mL (5/10) | 500 µL (AG 100-5-1.7) |
| | 500 µL PBS |
| AG 100-5-1.7, 25 µg/mL (25/100) | 250µL (AG 100-5-1.7) |
| | 750 µL PBS |

**Table 3**

| AG 100:14:1.7 (100 µg/mL) stock dispersion | |
|---|---|
| AG 100:14:1.7, 50 µg/mL (1/2) | 500 µL (AG 1000-14-1.7) |
| | 500 µL PBS |
| AG 100:14:1.7, 25 µg/mL (1/4) | 250 µL (AG 1000-14-1.7) |
| | 750 µL PBS |

### Bindzil 15/500 and Bindzil 159/500 reference material

The concentration of the reference dispersions will be 100 µg particles/mL and they will both be treated in the same way. A 1:2 dilution was first made by taking 500 µL of the stock dispersion and adding it to 500 µL PBS (0.01M, pH 7.4). A 1:4 dilution will be made by taking 500 µL of the 1:2 dilution and adding 500 µL PBS.

### Virus isolates

GMK cells and virus isolates will be treated and provided as described in Example 2.

### Acyclovir dilutions

Acyclovir dilutions will be prepared by from a stock solution into a set of acyclovir solutions having different concentrations of active compound (acyclovir).

### Method including assay of antiviral activity

The experiment will be performed as in Example 2, with the difference that in addition to the colloidal dispersions of silica nanoparticles referred to above, acyclovir solutions comprising different concentrations of acyclovir will be added to the cells for subsequent evaluation of a synergistic effect of the combination treatment on the viral activity. The possibility to reduce the dose of the anti-viral agent in the presence of AGSOL and still achieve the same antiviral effect will also be evaluated.

## Claims

1. A pharmaceutical composition comprising a colloidal dispersion of particles of silica having a particle size from 3 nm to 100 nm to which particles silver ions have been adsorbed, for use in a method of treatment of a viral infection.

2. The composition for use according to claim 1, wherein said particles of silica have a particle size of 3 to 25 nm.

3. The composition for use according to claim 1 or 2, wherein the silver ions are present in an amount of 0.05 ppm to 50 ppm by weight of the dispersion.

4. The composition for use according to any one of claims 1 to 3, wherein the silver ions are present in an amount of 0.5 ppm to 10 ppm by weight of the dispersion.

5. The composition for use according to any one of the preceding claims, wherein the particles comprise from 2 to 25 silver ions per silica particle.

6. The composition for use according to any one of claims 1 to 5, wherein the viral infection is an infection by a virus selected from the group consisting of the families Flaviviridae and Herpesviridae.

7. The composition for use according to any one of claims 1 to 6, wherein said virus is selected from the group consisting of the genuses Hepacivirus, Simplexvirus, Pegivirus, and Pestivirus.

8. The composition for use according to claim 7, wherein said virus is selected from the group consisting of: *Hepatitis virus C* (HCV), *Herpes simplex virus 1* (HSV-1), and *Herpes simplex virus 2* (HSV-2).

9. The composition for use according to claim 8, wherein said virus is *Hepatitis virus C* (HCV).

10. The composition for use according to claim 8, wherein said virus is *Herpes simplex virus 1* (HSV-1).

11. The composition for use according to claim 8, for the topical treatment of herpes labialis.

12. The composition for use according to any one of claims 1 to 11, wherein said composition further comprises one or more additional antiviral agent(s).

13. The composition for use according to claim 12, wherein said additional antiviral agent is an antiviral agent intended for the treatment or prevention of HSV-1, such as acyclovir or a functional equivalent thereof and/or an antiviral agent intended for the treatment or prevention of HCV, such as Boceprevir or a functional equivalent thereof.

14. The composition for use according to any one of claims 12 or 13, wherein said antiviral agent and composition are provided in a kit, said kit comprising:
a) a pharmaceutical formulation including an antiviral agent, optionally in admixture with a pharmaceutically acceptable excipient; and
b) a colloidal dispersion comprising particles of silica having a particle size from 3 nm to 100 nm to which particles silver ions have been adsorbed.

## Patentansprüche

1. Eine pharmazeutische Zusammensetzung, umfassend eine kolloidale Dispersion aus Siliciumdioxidteilchen mit einer Teilchengröße von 3 nm bis 100 nm, an denen Silberionenteilchen adsorbiert sind, zur Verwendung bei einem Verfahren zur Behandlung einer Virusinfektion.

2. Die Zusammensetzung zur Verwendung gemäß Anspruch 1, wobei die Siliciumdioxidteilchen eine Teilchengröße von 3 bis 25 nm aufweisen.

3. Die Zusammensetzung zur Verwendung gemäß Anspruch 1 oder 2, wobei die Silberionen in einer Menge von 0,05 ppm bis 50 ppm, bezogen auf das Gewicht der Dispersion, vorliegen.

4. Die Zusammensetzung zur Verwendung gemäß einem der Ansprüche 1 bis 3, wobei die Silberionen in einer Menge von 0,5 ppm bis 10 ppm, bezogen auf das Gewicht der Dispersion, vorliegen.

5. Die Zusammensetzung zur Verwendung gemäß einem der vorstehenden Ansprüche, wobei die Teilchen 2 bis 25 Silberionen pro Siliciumdioxidteilchen umfassen.

6. Die Zusammensetzung zur Verwendung gemäß einem der Ansprüche 1 bis 5, wobei die Virusinfektion eine Infektion durch ein Virus ist, ausgewählt aus der Gruppe bestehend aus den Familien Flaviviridae und Herpesviridae.

7. Die Zusammensetzung zur Verwendung gemäß einem der Ansprüche 1 bis 6, wobei das Virus ausgewählt ist aus der Gruppe, bestehend aus den Gattungen Hepacivirus, Simplexvirus, Pegivirus und Pestivirus.

8. Die Zusammensetzung zur Verwendung gemäß Anspruch 7, wobei das Virus ausgewählt ist aus der Gruppe, bestehend aus: *Hepatitis-C-Virus* (HCV), *Herpes-simplex-Virus 1* (HSV-1) und *Herpes-simplex-Virus 2* (HSV-2).

9. Die Zusammensetzung zur Verwendung gemäß Anspruch 8, wobei das Virus das *Hepatitis-C-Virus* (HCV) ist.

10. Die Zusammensetzung zur Verwendung gemäß Anspruch 8, wobei das Virus das *Herpes-simplex-Virus 1* (HSV-1) ist.

11. Die Zusammensetzung zur Verwendung gemäß Anspruch 8 zur topischen Behandlung von Herpes labialis.

12. Die Zusammensetzung zur Verwendung gemäß einem der Ansprüche 1 bis 11, wobei die Zusammensetzung ferner einen oder mehrere zusätzliche antivirale Wirkstoffe umfasst.

13. Die Zusammensetzung zur Verwendung gemäß Anspruch 12, wobei das zusätzliche antivirale Mittel ein antivirales Mittel ist, das zur Behandlung oder Vorbeugung von HSV-1 bestimmt ist, wie Aciclovir oder ein funktionelles Äquivalent davon, und/oder ein antivirales Mittel, das zur Behandlung oder Vorbeugung von HCV bestimmt ist, wie Boceprevir oder ein funktionelles Äquivalent davon.

14. Die Zusammensetzung zur Verwendung gemäß einem der Ansprüche 12 oder 13, wobei das antivirale Mittel und die Zusammensetzung in einem Kit bereitgestellt werden, wobei das Kit umfasst:
a) eine pharmazeutische Formulierung, die ein antivirales Mittel enthält, gegebenenfalls in Beimischung mit einem pharmazeutisch verträglichen Hilfsstoff; und
b) eine kolloidale Dispersion, umfassend Siliciumdioxidteilchen mit einer Teilchengröße von 3 nm bis 100 nm, an denen Silberionen adsorbiert wurden.

## Revendications

1. Composition pharmaceutique comportant une dispersion colloïdale de particules de silice ayant une taille de particule de 3 nm à 100 nm sur laquelle des particules d'ions argent ont été adsorbées, pour une utilisation dans un procédé de traitement d'une infection virale.

2. Composition pour une utilisation selon la revendication 1, dans laquelle lesdites particules de silice ont une taille de particule de 3 à 25 nm.

3. Composition pour une utilisation selon la revendication 1 ou 2, dans laquelle les ions argent sont présents en une quantité de 0,05 ppm à 50 ppm en poids de la dispersion.

4. Composition pour une utilisation selon l'une quelconque des revendications 1 à 3, dans laquelle les ions argent sont présents en une quantité de 0,5 ppm à 10 ppm en poids de la dispersion.

5. Composition pour une utilisation selon l'une quelconque des revendications précédentes, dans laquelle les particules comportent de 2 à 25 ions argent par particule de silice.

6. Composition pour une utilisation selon l'une quelconque des revendications 1 à 5, dans laquelle l'infection virale est une infection par un virus choisi dans le groupe constitué des familles des Flaviviridae et des Herpesviridae.

7. Composition pour une utilisation selon l'une quelconque des revendications 1 à 6, dans laquelle ledit virus est choisi dans le groupe constitué des genres hépacivirus, simplexvirus, pegivirus et pestivirus.

8. Composition pour une utilisation selon la revendication 7, dans laquelle ledit virus est choisi dans le groupe consistant en : le *virus de l'hépatite C* (VHC), le *virus herpès simplex 1* (VHS-1) et le *virus herpès simplex 2* (VHS-2).

9. Composition pour une utilisation selon la revendication 8, dans laquelle ledit virus est le *virus de l'hépatite C* (VHC).

10. Composition pour une utilisation selon la revendication 8, dans laquelle ledit virus est le *virus herpès simplex 1* (VHS-1).

11. Composition pour une utilisation selon la revendication 8, pour le traitement topique de l'herpès labial.

12. Composition pour une utilisation selon l'une quelconque des revendications 1 à 11, dans laquelle ladite composition comporte en outre un ou plusieurs agents antiviraux supplémentaires.

13. Composition pour une utilisation selon la revendication 12, dans laquelle ledit agent antiviral supplémentaire est un agent antiviral destiné au traitement ou à la prévention du VHS-1, tel que l'aciclovir ou un équivalent fonctionnel de celui-ci et/ou un agent antiviral destiné au traitement ou à la prévention du VHC, tel que le Boceprevir ou un équivalent fonctionnel de celui-ci.

14. Composition pour une utilisation selon l'une quelconque des revendications 12 ou 13, dans laquelle ledit agent antiviral et ladite composition sont fournis dans un kit, ledit kit comportant :
a) une formulation pharmaceutique incluant un agent antiviral, facultativement en mélange avec un excipient pharmaceutiquement acceptable ; et
b) une dispersion colloïdale comportant des particules de silice ayant une taille de particule de 3 nm à 100 nm sur lesquelles des particules d'ions argent ont été adsorbées.
